# EUROPEAN PATENT SPECIFICATION

(11) **EP 1 714 653 B1**
(45) Date of publication and mention of the grant of the patent: **24.11.2010**
(21) Application number: 05709954.1
(22) Date of filing: 09.02.2005
(51) Int. Cl.: A61K 31/4745, A61K 9/08, A61K 47/04, A61K 47/10, A61K 47/12, A61K 47/22, A61P 35/00

(54) **AQUEOUS SOLUTION PREPARATION CONTAINING A CAMPTOTHECIN**
WÄSSRIGE LÖSUNG MIT EINEM CAMPTOTHECIN
PREPARATION DE SOLUTION AQUEUSE CONTENANT UNE CAMPTOTHECINE

(30) Priority: 13.02.2004 JP 2004035985; 13.02.2004 JP 2004035986
(43) Date of publication of application: 25.10.2006
(73) Proprietor: KABUSHIKI KAISHA YAKULT HONSHA, Minato-ku, Tokyo 105-8660 (JP)
(72) Inventor: NAKAZAWA, Masako, KABUSHIKI KAISHA YAKULT HONSHA, Tokyo 1058660 (JP); AIYAMA, Ritsuo, KABUSHIKI KAISHA YAKULT HONSHA, Tokyo 1058660 (JP)
(74) Representative: Hartz, Nikolai
(86) International application number: PCT/JP2005/001902
(87) International publication number: WO 2005/077370

(56) References cited:
- WO-A-03/086471
- JP-A- 2004 277 374
- JP-B2- 3 004 077
- US-A- 5 674 874
- US-B1- 6 476 043
- US-B1- 6 653 319

## Description

### Technical Field

This invention relates to a stable aqueous solution preparation which has excellent solubility for camptothecins.

### Background Art

Camptothecin (CPT) is an alkaloid found in fruits and roots of happy tree (camptotheca acuminata) from China. 7-ethyl-10-piperidinopiperidinocarbonyloxycamptothecin (CPT-11) (Patent Document 1) which is a semisynthetic derivative of the camptothecin is an important compound since it has the high antitumor activity of the camptothecin simultaneously with reduced toxicity. This 7-ethyl-10-piperidinopiperidinocarbonyloxycamptothecin is metabolized in the living body to produce 7-ethyl-10-hydroxycamptothecin (SN-38) which is a semisynthetic derivative exhibiting the activity (Patent Document 2).

Administration of camptothecins such as 7-ethyl-10-piperidinopiperidinocarbonyloxycamptothecin is mainly conducted by intravenous injection. Therefore, camptothecins such as 7-ethyl-10-piperidinopiperidinocarbonyloxycamptothecin are currently commercially available and used as a preparation which has been isotonized with sorbitol or the like. Various attempts have been made to produce preparations of the camptothecins, and exemplary such attempts are a controlled release preparation wherein a camptothecin derivative is incorporated in a copolymer of collagen and 2-hydroxyethyl methacrylate (Patent Document 3) and a controlled release preparation wherein camptothecin or its derivative in a carrier comprising a copolymer of polylactic acid and glycolic acid copolymer (Patent Document 4).
However, camptothecins exhibit low solubility in water, and heating is required in preparing an aqueous solution preparation, and there is a demand for the development of an aqueous solution preparation containing camptothecins which can be produced in a simplified manner without requiring such heating.
[Patent Document 1] Japanese Patent Publication No. 1991-4077
[Patent Document 2] Japanese Patent Publication No. 1987-47193
[Patent Document 3] Japanese Patent Application Laid-Open No. 1995-277981
[Patent Document 4] Japanese Patent Application Laid-Open No. 1998-17472
US 6,476,043 B1 describes a medicinal product with reduced gastrointestinal toxicity, comprising a solution of a camptothecin derivative and a solution consisting essentially of sodium chloride.
JP 2004 277374 A describes an aqueous solution preparation containing camptothecins and excipients.

### Disclosure of the Invention

### Problems to Be Solved by the Invention

An obj ect of the present invention is to provide an aqueous solution preparation containing camptothecins which does not require heating in its production, and wherein camptothecins have been solubilized in a stable manner.

### Means for Solving the Problems

In view of the situation as described above, the inventors of the present invention made an intensive study and found that, when acetic acid and sodium acetate are incorporated in the aqueous solution preparation containing the camptothecins, and the aqueous solution preparation is adjusted to a particular pH range, solubility of the camptothecins in the aqueous solution increases, and a stable aqueous solution preparation containing camptothecins having a solubility for camptothecins higher than conventional products can be obtained. The present invention has been completed on the bases of such finding.

Accordingly, the present invention provides an aqueous solution preparation containing a camptothecin, wherein
the preparation comprises the following components (A) and (B):
(A) the camptothecin defined in claim 1, and
(B) acetic acid and sodium acetate, and
the preparation is at a pH of 2 to 5.

### Merits of the Invention

In the case of the aqueous solution preparation of the present invention, the camptothecin can be dissolved at a high concentration without requiring heating in the production process.

### Best Modes for Carrying out the Invention

The component (A) is the effective component in the aqueous solution preparation of the present invention. This camptothecin is 7-ethyl-10-piperidinopiperidinocarbonyloxycamptothecin (hereinafter sometimes referred as CPT-11).

The sodium acetate in the component (B) used in the aqueous solution preparation of the present invention may be generated by adding acetic acid and alkaline agent in the aqueous solution preparation. Exemplary alkaline agents used in such case include sodium hydroxide, sodium carbonate, and sodium hydrogencarbonate, and use of sodium hydroxide is preferable. Alternatively, sodium acetate may be generated in the aqueous solution preparation by salt exchange with another compound.

The aqueous solution preparation of the present invention preferably contains the component (B), namely, acetic acid and sodium acetate at a content of 0.1 to 10% by weight in terms of acetic acid.
The content of the acetic acid and sodium acetate in terms of acetic acid per 100 mg of the camptothecin in the aqueous solution preparation of the present invention is preferably in the range of 10 to 2000 mg, more preferably 10 to 1000 mg, and most preferably 20 to 500 mg in view of improving solubility of the camptothecin in the aqueous solution preparation.

Further incorporation in the aqueous solution preparation of the present invention of the component (C), namely, (i) cyclodextrin, (ii) ascorbic acid and sodium ascorbate, (iii) propylene glycol, or (iv) at least one compound selected from the group consisting of sodiumhydrogen sulfite, sodium sulfite, potassium pyrosulfite, sodium erythorbate, sodium thioglycolate, sodium pyrosulfite, and α-thioglycerin is preferable since such incorporation improves solubility of the camptothecin in the preparation.

The cyclodextrin (i) of component (C) is a irreducible maltooligosaccharide comprising 6 to 12 glucose molecules which have been linked in cycle by α-1,4 glycosidic linkage, and examples include α-cyclodextrin, β-cyclodextrin, γ-cyclodextrin, and derivatives thereof. Exemplary cyclodextrin derivatives include maltosyl cyclodextrin, glycosyl cyclodextrin, dimethyl cyclodextrin, and hydroxypropyl cyclodextrin. Preferable examples of the cyclodextrin include β-cyclodextrin, γ-cyclodextrin, and hydroxypropyl β-cyclodextrin.

The aqueous solution preparation of the present invention preferably contains the cyclodextrin at a content of 1 to 20% by weight, and in particular, at 1.5 to 14% by weight in view of improving the solubility of the camptothecin.
In view of improving solubility of the camptothecin in the aqueous solution preparation, content of the cyclodextrin per 100 mg of the camptothecin in the aqueous solution preparation of the present invention is preferably in the range of 30 to 1000 mg, and in particular, 90 to 700 mg.
When the cyclodextrin is used, the content of the component (B), namely, the acetic acid and the sodium acetate is preferably used at a content in terms of acetic acid of 0.1 to 5.0% by weight, more preferably 0.3 to 3.0% by weight, and most preferably 0.5 to 2.0% by weight in view of improving solubility of the camptothecin.

Incorporation in the aqueous solution preparation of the present invention of component (C) (ii) ascorbic acid and sodium ascorbate is preferable since the resulting aqueous solution preparation will exhibit an improved solubility of the camptothecin.

The sodium ascorbate may be generated by adding an alkaline agent to the ascorbic acid in the aqueous solution preparation. Exemplary alkaline agents used in such case include sodium hydroxide, sodium carbonate, and sodium hydrogencarbonate, and use of sodium hydroxide is preferable. Alternatively, sodium ascorbate may be generated in the aqueous solution preparation by salt exchange with another compound.

The aqueous solution preparation of the present invention preferably contains the ascorbic acid and the sodium ascorbate at a content in terms of ascorbic acid of 5 to 20% by weight, and in particular, 6 to 15% by weight.
When the ascorbic acid and the sodium ascorbate are used, the component (B), namely, the acetic acid and the sodium acetate is preferably used at a content in terms of the acetic acid of 0.5 to 8% by weight, and in particular, at 0.7 to 6% by weight in view of the solubility of the camptothecin.

The acetic acid, ascorbic acid, and their sodium salts are preferably incorporated at a total content in terms of the respective acids of 0.1 to 20% by weight, more preferably 0.3 to 15% by weight, and most preferably 0.4 to 14% by weight in view of the solubility of the camptothecin.
The acetic acid, ascorbic acid, and their sodium salts are preferably incorporated at a total content in terms of the respective acids of 500 to 2000 mg, and in particular, 800 to 1500 mg per 100 mg of the camptothecin in the aqueous solution preparation of the present invention in view of improving solubility of the camptothecin in the aqueous solution preparation.

When propylene glycol (iii) is used for the component (C), it is preferably incorporated in the aqueous solution preparation of the present invention at a content of 40 to 70% by weight, and in particular at 50 to 60% by weight.
The propylene glycol is preferably incorporated at a content of 1 to 4 g, and in particular, at 2 to 3 g per 100 mg of the camptothecin in the aqueous solution preparation of the present invention in view of improving solubility of the camptothecin in the aqueous solution preparation.
When propylene glycol is used, the content of the component (B), namely, acetic acid and sodium acetate in terms of acetic acid is preferably in the range of 0.5 to 8% by weight, and more preferably 0.7 to 6% by weight in view of improving solubility of the camptothecin.

Incorporation in the aqueous solution preparation of the present invention of component (C) (iv) at least one compound selected from the group consisting of sodium hydrogen sulfite, sodium sulfite, potassium pyrosulfite, sodium erythorbate, sodium thioglycolate, sodium pyrosulfite, and α-thioglycerin is preferable since the resulting aqueous solution preparation will exhibit an improved solubility of the camptothecin.

The compound selected from the component (C)(iv) is preferably incorporated in the aqueous solution preparation of the present invention at a content of 1 to 300mg, and in particular, at a content of 10 to 200 mg per 100 mg of the camptothecins in view of improving solubility of the camptothecin.

The aqueous solution preparation of the present invention is preferably at pH 2 to 5, and more preferably at 2.5 to 4.8 at room temperature (25°C) in view of improving the solubility of the camptothecin. The pH is preferably adjusted by using an acid such as acetic acid, hydrochloric acid, and sulfuric acid, or a sodium-containing alkali such as sodium hydroxide, sodium carbonate, and sodium hydrogencarbonate.

The aqueous solution preparation of the present invention is useful as an antitumor preparation since the camptothecin which is the effective component has excellent therapeutic effects for malignant tumors. Exemplary applicable malignant tumors include lung cancer, uterine cancer, ovarian cancer, gastric cancer, colorectal cancer, breast cancer, lymphoma, and pancreatic cancer.

Preferable dosage formof the aqueous solution preparation of the present invention is preparation for injection, and in particular, preparation for intravenous administration. In preparing such preparation for injection, the preparation may contain in addition to the camptothecin additives such as distilled water for injection, sugars as represented by glucose, mannose, and lactose, inorganic salts as represented by sodium chloride, an organic amine such as HEPES and PIPES, and components normally employed in an injection such as stabilizer, excipient, and buffer. The camptothecin is preferably incorporated in the injection preparation at an amount of 1 to 50 mg/mL, and in particular, at an amount of 10 to 30 mg/mL.

### Example

The present invention will be described further in detail with examples; however, it should not be construed that the present invention is limited thereto.

### Example 1

Acetic acid was added to the aqueous solution shown in Table 1 for pH adjustment, and to 10 mL of this solution was added 250 to 500 mg of CPT-11. The mixture was ultrasonicated for 10 minutes for dispersion and dissolution of the CPT-11 in the aqueous solution, and stirred at room temperature for the period indicated in Table 1. Next, the solution was aliquoted, and centrifuged at 3000 r/min for 30 minutes, and the supernatant was filtered through a 0.45 µm filter. 1 mL of the filtrate was accurately measured, and made up to 50 mL with 90% methanol aqueous solution. The amount of CPT-11 in the solution was measured by HPLC under the conditions as described below.

HPLC conditions:
Column: Symmetry Shield RP18 (3.5 µm, 4. 6 x 50 mm)
Column temperature: 50°C
Flow rate: 2.0 mL/min
Mobile phase: solution A (50 mmol/L formate buffer (pH 5.5) / acetonitrile / methanol = 850 / 100 / 50) and solution B (50 mmol/L formate buffer (pH 5.5) / acetonitrile / methanol = 750 / 250 / 50). Linear gradient of solution B of 0 to 100% in 15 minutes, followed by 5 minute equilibration with 100% solution A.
Amount injected: 10µL
Detection wavelength: 254nm

The measurement results for the amount of CPT-11 in each aqueous solution after stirring for 1 or 2 days at room temperature are shown in Table 1. The results are shown in the amount of CPT-11 in 1 mL of the aqueous solution (CPT-11 in mg/mL).

**Table 1**

| | Amount (mg) of the components added per 5 mL of the aqueous solution | | | pH^{*1} of the aqueous solution | Period of stirring | |
|---|---|---|---|---|---|---|
| | Sodium acetate | Type of the cyclodextrin | | | 1 day | 2 day |
| Examples of the present invention | | | | | | |
| 1 | 100 | - | | 4.0 | 28.07 | 27.87 |
| 2 | 100 | β | 92.5 | 4.0 | 29.46 | 29.13 |
| 3 | 100 | γ | 336 | 4.0 | 31.66 | 31.52 |
| 4 | 100 | γ | 672 | 4.0 | 30.89 | 32.09 |
| 5 | 100 | Hydroxypropyl β 308 | | 4.0 | 31.12 | 31.18 |

| Comparative Examples | | | | | | |
|---|---|---|---|---|---|---|
| 1 | Lactic acid | | 250 | 4.0 | - | 19.03 |
| 2 | Malic acid | | 250 | 4.0 | - | 18.89 |
| 3 | Citric acid | | 250 | 4.0 | - | 20.06 |

| | | | | | | |
|---|---|---|---|---|---|---|
| *1 pH was adjusted by adding acetic acid. | | | | | | |

All of the aqueous solution preparations containing the camptothecin according to the present invention exhibited excellent solubility for CPT-11. These aqueous solution preparations also exhibited no color change or crystal precipitation when left at room temperature (25°C) for 3 days with no shading. In addition, no precipitation of CPT-11 crystals was noted after shaking of the preparations.

### Example 2

To 10 mL of the aqueous solution shown in Table 2 was added 250 to 500 mg of CPT-11. The mixture was ultrasonicated for 10 minutes for dispersion and dissolution of the CPT-11 in the aqueous solution, and stirred at room temperature for the period indicated in Table 2. Next, the solution was aliquoted, and centrifuged at 3000 r/min for 30 minutes, and the supernatant was filtered through a 0.45 µm filter. 1 mL of the filtrate was accurately measured, and made up to 50 mL with 90% methanol aqueous solution. The amount of CPT-11 in the solution was measured by HPLC under the same conditions as Example 1.

The measurement results for the amount of CPT-11 in each aqueous solution after stirring for 1 or 2 days at room temperature are shown in Table 2. The results are shown in the amount of CPT-11 in 1 mL of the aqueous solution (CPT-11 in mg/mL).

**Table 2**

| No. | Amount (mg) of the components added per 5 mL of the aqueous solution | | | | pH of the aqueous solution | Period of stirring | |
|---|---|---|---|---|---|---|---|
| | Acetic acid | Sodium acetate | Ascorbic acid | NaOH | | 1 day | 2 day |
| Examples of the present invention | | | | | | | |
| 6 | 200 | 20 | 700 | | 2.8 | 44.54 | 44.61 |
| 7 | 200 | 20 | 500 | | 2.9 | 38.70 | 38.45 |
| 8 | 200 | 50 | 700 | | 3.2 | 43.64 | 44.07 |
| 9 | 200 | 100 | 700 | | 3.6 | 45.87 | 47.24 |
| 10 | 200 | 20 | 700 | 15 | 3.1 | 43.45 | 43.56 |
| 11 | 200 | 20 | 700 | 20 | 3.2 | 41.97 | 41.79 |
| 12 | 200 | 20 | 700 | 50 | 3.7 | 42.41 | 42.50 |
| 13 | 200 | 20 | 700 | 100 | 4.2 | 38.16 | 39.11 |

| Comparative Example | | | | | | | |
|---|---|---|---|---|---|---|---|
| 4 | | | 250 | | 4.0^{*2} | - | 20.91 |

| | | | | | | | |
|---|---|---|---|---|---|---|---|
| *2: pH was adjusted by adding sodium hydroxide. | | | | | | | |

The aqueous solution preparations containing the camptothecin according to the present invention of Nos. 6 to 13 exhibited excellent solubility for CPT-11. These aqueous solution preparations also exhibited no color change or crystal precipitation when left at room temperature (25°C) for 3 days with no shading. In addition, no precipitation of CPT-11 crystals was noted after shaking of the preparations. On the other hand, the preparation containing only ascorbic acid exhibited insufficient solubility.

### Example 3

Acetic acid was added to the aqueous solution shown in Table 3 for pH adjustment to 4.0, and to 10 mL of this solution was added 250 to 500 mg of CPT-11. The mixture was ultrasonicated for 10 minutes for dispersion and dissolution of the CPT-11 in the aqueous solution, and stirred at room temperature for the period indicated in Table 3. Next, the solution was aliquoted, and centrifuged at 3000 r/min for 30 minutes, and the supernatant was filtered through a 0.45 µm filter. 1 mL of the filtrate was accurately measured, and made up to 50 mL with 90% methanol aqueous solution. The amount of CPT-11 in the solution was measured under the same conditions as Example 1.

The measurement results for the amount of CPT-11 in each aqueous solution after stirring for 1 or 2 days at room temperature are shown in Table 3. The results are shown in the amount of CPT-11 in 1 mL of the aqueous solution (CPT-11 in mg/mL).

**Table 3**

| No. | Amount (mg) of the components added per 5 mL of the aqueous solution | | pH of the aqueous solution *3 | Period of stirring | |
|---|---|---|---|---|---|
| | Sodium acetate | Component added | | 1 day | 2 day |
| Examples of the present invention | | | | | |
| 14 | 30 | Sodium hydrogen sulfite 200 | 4.0 | 25.38 | 25.30 |
| 15 | 30 | Sodium sulfite 100 | 4.0 | 27.93 | 27.82 |
| 16 | 30 | Sodium thioglycolate 50 | 4.0 | 22.15 | 22.38 |
| 17 | 30 | Potassium pyrosulfite 200 | 4.0 | 25.70 | 25.79 |
| 18 | 30 | Sodium pyrosulfite 50 | 4.0 | 21.80 | 21.97 |
| 19 | 30 | α-thioglycerin 50 | 4.0 | 19.63 | 20.32 |
| 20 | 30 | Sodium erythorbate 50 | 4.0 | 21.99 | 21.69 |

| | | | | | |
|---|---|---|---|---|---|
| *3 pH was adjusted by adding acetic acid. | | | | | |

The aqueous solution preparations containing the camptothecin according to the present invention of Nos. 14 to 20 exhibited excellent solubility for CPT-11. These aqueous solution preparations also exhibited no color change or crystal precipitation when left at room temperature (25°C) for 3 days with no shading. In addition, no precipitation of CPT-11 crystals was noted after shaking of the preparations.

### Example 4

To 10 mL of aqueous solution at pH 4.0 containing 100 mg of sodium acetate, 20 mg of acetic acid, 60 mg of sodium sulfite, and 3000 mg of propylene glycol was added 250 to 500 mg of CPT-11, and the mixture was ultrasonicated for 10 minutes for dispersion anddissolutionof the CPT-11 in the aqueous solution. The amount of CPT-11 per 1 mL of the aqueous solution (CPT-11, mg/mL) was then measured by repeating the procedure of Example 1, and the amount was 32.26 mg/mL at day 1 and 31.20 mg/mL at day 2. In the comparative solution containing no sodium acetate, acetic acid, or sodium sulfite, the amount of CPT-11 dissolved was 18.46 mg/mL at day 1, and 18.12 mg/mL at day 2.

### Example 5

The following injection preparations 1 to 7 were obtained by the procedure as described below.
To 3.5 mL of the solution having various additives preliminarily dissolved therein was added 100 mg of irinotecan hydrochloride (CPT-11), and the mixture was thoroughly stirred to dissolve the irinotecan hydrochloride. To this solution was added the solution having various additives preliminarily dissolved therein to the total volume of 5 mL.

### Preparation 1

| | |
|---|---|
| Irinotecan hydrochloride | 100 mg |
| Sodium acetate | 50 mg |
| Acetic acid | 120 mg |
| Water for injection | 5 mL in total |
| pH | 4.0 |

### Preparation 2

| | |
|---|---|
| Irinotecan hydrochloride | 100 mg |
| Sodium acetate | 100 mg |
| Acetic acid | 380 mg |
| β cyclodextrin | 92.5 mg |
| Water for injection | 5 mL in total |
| pH | 4.0 |

### Preparation 3

| | |
|---|---|
| Irinotecan hydrochloride | 100 mg |
| Acetic acid | 380 mg |
| Sodium hydroxide | 46 mg |
| γ cyclodextrin | 672 mg |
| Water for injection | 5 mL in total |
| pH | 4.0 |

### Preparation 4

| | |
|---|---|
| Irinotecan hydrochloride | 100 mg |
| Ascorbic acid | 700 mg |
| Sodium acetate | 100 mg |
| Acetic acid | 200 mg |
| Water for injection | 5 mL in total |
| pH | 3.6 |

### Preparation 5

| | |
|---|---|
| Irinotecan hydrochloride | 100 mg |
| Sodium acetate | 20 mg |
| Sodium ascorbate | 700 mg |
| Acetic acid | 200 mg |
| Water for injection | 5 mL in total |
| pH | 4.5 |

### Preparation 6

| | |
|---|---|
| Irinotecan hydrochloride | 100 mg |
| Sodium ascorbate | 20 mg |
| Sodium hydroxide | 100 mg |
| Acetic acid | 20 mg |
| Water for injection | 5 mL in total |
| pH | 4.5 |

### Preparation 7

| | |
|---|---|
| Irinotecan hydrochloride | 100 mg |
| Sodium acetate | 30 mg |
| Acetic acid | 100 mg |
| Sodium sulfite | 100 mg |
| Water for injection | 5 mL in total |
| pH | 4.0 |

The aqueous solution preparations containing the camptothecin (injections) of Preparations 1 to 7 were pale yellow transparent aqueous solutions, and precipitation of the irinotecan hydrochloride crystals was noted in none of the solutions.

## Claims

1. An aqueous solution preparation containing 7-ethyl-10-piperidinopiperidinocarbonyloxycamptothecin, wherein
the preparation comprises the following components (A) and (B):
(A) 7-ethyl-10-piperidinopiperidinocarbonyloxycamptothecin, and
(B) acetic acid and sodium acetate, and the preparation is at a pH of 2 to 5.

2. The aqueous solution preparation containing 7-ethyl-10-piperidinopiperidinocarbonyloxycamptothecin according to claim 1, wherein
the preparation further comprises component (C):
(C)
(i) cyclodextrin,
(ii) ascorbic acid and sodium ascorbate,
(iii) propylene glycol, or
(iv) at least one compound selected from the group consisting of sodium hydrogen sulfite, sodium sulfite, potassium pyrosulfite, sodium erythorbate, sodium thioglycolate, sodium pyrosulfite, and α-thioglycerin.

3. The aqueous solution preparation containing 7-ethyl-10-piperidinopiperidinocarbonyloxycamptothecin according to claim 1 or 2, wherein the aqueous solution preparation is an antitumor preparation.

4. The aqueous solution preparation containing 7-ethyl-10-piperidinopiperidinocarbonyloxycamptothecin according to any one of claims 1,2 or 3, wherein the aqueous solution preparation is a preparation for injection.

## Patentansprüche

1. Präparat in wässriger Lösung enthaltend 7-Ethyl-10-piperidinopiperidino-carbonyloxycamptothecin, wobei das Präparat die folgenden Komponenten (A) und (B) enthält:
(A) 7-Ethyl-10-piperidinopiperidinocarbonyloxycamptothecin, und
(B) Essigsäure und Natriumacetat und
das Präparat einen pH von 2 bis 5 aufweist.

2. Das Präparat in wässriger Lösung enthaltend 7-Ethyl-I10-piperidinopiperidino-carbonyloxycamptothecin gemäß Anspruch 1, worin das Präparat ferner die folgende Komponente (C) enthält:
(C)
(i) Cyclodextrin,
(ii) Ascorbinsäure und Natriumascorbat,
(iii) Propylenglycol oder
(iv) mindestens eine Verbindung ausgewählt aus der Gruppe bestehend aus Natriumhydrogensulfit, Natriumsulfit, Kaliumpyrosulfit, Natriumerythorbat, Natriumthioglycolat, Natriumpyrosulfit, und α-Thioglycerin.

3. Das Präparat in wässriger Lösung enthaltend 7-Ethyl-10-piperidinopiperidino-carbonyloxycamptothecin gemäß Anspruch 1 oder 2, worin das Präparat in wässriger Lösung ein Antitumor-Präparat ist.

4. Das Präparat in wässriger Lösung enthaltend 7-Ethyl-10-piperidinopiperidino-carbonyloxycamptothecin gemäß einem der Ansprüche 1, 2 oder 3, worin das Präparat in wässriger Lösung ein Präparat für Injektionen ist.

## Revendications

1. Préparation de solution aqueuse contenant de la 7-éthyl-10-pipéridinopipéridinocarbonyloxycamptothécine, laquelle préparation comprend les composants (A) et (B) suivants :
(A) 7-éthyl-10-pipéridinopipéridinocarbonyloxy-camptothécine, et
(B) acide acétique et acétate de sodium, et
laquelle préparation a un pH de 2 à 5.

2. Préparation de solution aqueuse contenant de la 7-éthyl-10-pipéridinopipéridinocarbonyloxycamptothécine selon la revendication 1, laquelle préparation comprend en outre le composant (C) :
(C)
(i) cyclodextrine,
(ii) acide ascorbique et ascorbate de sodium, (iii)propylèneglycol, ou
(iv) au moins un composé choisi dans l'ensemble constitué par l'hydrogénosulfite de sodium, le sulfite de sodium, le pyrosulfite de potassium, l'érythorbate de sodium, le thioglycolate de sodium, le pyrosulfite de sodium, et l'α-thioglycérine.

3. Préparation de solution aqueuse contenant de la 7-éthyl-10-pipéridinopipéridinocarbonyloxycamptothécine selon la revendication 1 ou 2, laquelle préparation en solution aqueuse est une préparation antitumorale.

4. Préparation de solution aqueuse contenant de la 7-éthyl-10-pipéridinopipéridinocarbonyloxycamptothécine selon l'une quelconque des revendications 1, 2 et 3, laquelle préparation en solution aqueuse est une préparation injectable.
